# EUROPEAN PATENT APPLICATION

(11) **EP 4 014 901 A2**
(43) Date of publication of application: **22.06.2022**
(21) Application number: 22156104.6
(22) Date of filing: 15.05.2020
(51) Int. Cl.: A61B 17/32, A61B 17/42, A61B 17/3207

(54) **MANUALLY-ACTUATED TISSUE RESECTING INSTRUMENTS**

(30) Priority: 17.05.2019 US 201962849183 P
(62) Divisional of application: 20175092.4
(71) Applicant: Covidien LP, Mansfield, MA 02048 (US)
(72) Inventor: BEGG, Nikolai D., Wellesley, 02481 (US); LAURITO, Tyler J., Boston, 02210 (US)
(74) Representative: Zimmermann & Partner Patentanwälte mbB

(57) **Abstract**

A tissue resecting instrument includes a housing, an outer shaft extending distally from the housing and defining a window at a distal end potion thereof, an inner cutting shaft extending through the outer shaft, a drive assembly, a trigger, and an adjustable resistance mechanism. The inner cutting shaft is translatable and/or rotatable relative to the outer shaft. The drive assembly is coupled to the inner cutting shaft and configured to drive the translation and/or rotation thereof. Manual actuation of the trigger actuates the drive assembly to drive the translation and/or rotation of the inner cutting shaft. The adjustable resistance mechanism includes a spring coupled between the trigger and the housing, and a control knob operably coupled to the spring to enable adjustment of a resistance to pivoting of the trigger from an un-actuated position to an actuated position.

## Description

### FIELD

The present disclosure relates generally to the field of tissue resection. In particular, the present disclosure relates to manually-actuated tissue resecting instruments.

### BACKGROUND

Tissue resecting instruments are commonly used in endoscopic tissue resection procedures within an organ, such as a uterus, by inserting an endoscope (or hysteroscope) into the uterus and passing the tissue resection instrument through the endoscope (or hysteroscope) and into the uterus. With respect to such endoscopic tissue resection procedures, tissue is resected at the surgical site and suctioned proximally through the tissue resecting instrument, along with fluid at the surgical site.

### SUMMARY

As used herein, the term "distal" refers to the portion that is described which is further from a user, while the term "proximal" refers to the portion that is being described which is closer to a user. Further, any or all of the aspects described herein, to the extent consistent, may be used in conjunction with any or all of the other aspects described herein.

Provided in accordance with aspects of the present disclosure is a tissue resecting instrument including a housing, an outer shaft extending distally from the housing and defining a window at a distal end potion thereof, an inner cutting shaft extending through the outer shaft, a drive assembly coupled to the inner cutting shaft, a trigger coupled to the drive assembly, and an adjustable resistance mechanism. The inner cutting shaft is translatable and/or rotatable relative to the outer shaft to cut tissue extending through the window. The drive assembly is configured to drive the translation and/or rotation of the inner cutting shaft. Manual actuation of the trigger actuates the drive assembly to drive the translation and/or rotation of the inner cutting shaft. The adjustable resistance mechanism includes a spring coupled between the trigger and the housing, and a control knob operably coupled to the spring to enable adjustment of a resistance to pivoting of the trigger from an un-actuated position to an actuated position.

In an aspect of the present disclosure, the spring is a coil spring and adjustment of the resistance to pivoting includes adjusting an initial length of the coil spring. In such aspects, the coil spring may be coupled at a first end portion thereof to the trigger and a second end portion thereof may be movable to adjust the initial length of the coil spring.

In another aspect of the present disclosure, the spring is a torsion spring whereby adjustment of the resistance to pivoting includes adjusting a relative positioning of first and second legs of the torsion spring. In such aspects, the second leg may be fixed relative to the housing and the first leg is rotatable about a body of the torsion spring and relative to the second leg to adjust the relative positioning of the first and second legs.

In still another aspect of the present disclosure, adjustment of the resistance to pivoting adjusts an initial resistance to the pivoting of the trigger from the un-actuated position to the actuated position.

In yet another aspect of the present disclosure, adjustment of the resistance to pivoting adjusts a resistance profile through at least a portion of the pivoting of the trigger from the un-actuated position to the actuated position.

In still yet another aspect of the present disclosure, a vacuum generator is coupled to the drive assembly and the inner cutting shaft such that, during at least a portion of the manual actuation of the trigger to drive the drive assembly, the vacuum generator generates vacuum to suction cut tissue through the inner cutting shaft and into the vacuum generator. In such aspects, a tissue collection cartridge configured to releasably engage the housing may be provided. The tissue collection cartridge defines a port configured to communicate with the vacuum generator such that, during at least a portion of the manual actuation of the trigger to drive the drive assembly, cut tissue is urged from the vacuum generator through the port into the tissue collection cartridge.

In another aspect of the present disclosure, the trigger is pivotably coupled to the housing and movable relative thereto between the un-actuated position and the actuated position to drive the drive assembly to translate the inner cutting shaft between a more-proximal position and a more-distal position.

In another aspect of the present disclosure, the spring is further configured to bias the trigger towards the un-actuated position.

Another tissue resecting instrument provided in accordance with aspects of the present disclosure includes an end effector assembly, a handpiece assembly, and an isolation mechanism. The end effector assembly includes a proximal hub, an outer shaft extending distally from the proximal hub and defining a window at a distal end potion thereof, and an inner cutting shaft extending through the outer shaft. The inner cutting shaft is translatable and/or rotatable relative to the outer shaft to cut tissue extending through the window. The handpiece assembly includes a housing, a drive assembly, and a trigger. The drive assembly is disposed within the housing, coupled to the inner cutting shaft, and configured to drive the translation and/or rotation of the inner cutting shaft. The trigger is coupled to the drive assembly such that manual actuation of the trigger actuates the drive assembly to drive the translation and/or rotation of the inner cutting shaft. The isolation mechanism is operably coupled between the proximal hub and the housing to engage the proximal hub and the housing with one another. The isolation mechanism isolates movement of the housing from the end effector assembly through a range of motion.

In an aspect of the present disclosure, the isolation mechanism includes a ball and socket joint. In such aspects, one of the proximal hub or the housing may include a concave surface defining a socket of the ball and socket joint, and the other of the proximal hub or the housing may include a convex surface defining a ball of the ball and socket joint. The ball is received within the socket.

In another aspect of the present disclosure, the isolation mechanism includes a bellows. In such aspects, a proximal end portion of the bellows may be coupled to the housing while a distal end portion of the bellows may be coupled to the proximal hub.

In still another aspect of the present disclosure, a connector operably couples the inner cutting shaft and the drive assembly with one another.

In yet another aspect of the present disclosure, the connector is configured to enable the drive assembly to drive the translation and/or rotation of the inner cutting shaft in each of an aligned position, a yawed position, and a pitched position of the housing relative to the proximal hub.

In still yet another aspect of the present disclosure, the handpiece assembly further includes a vacuum generator coupled to the drive assembly and the inner cutting shaft such that, during at least a portion of the manual actuation of the trigger to drive the drive assembly, the vacuum generator generates vacuum to suction cut tissue through the inner cutting shaft and into the vacuum generator.

In another aspect of the present disclosure, the handpiece assembly further includes a tissue collection cartridge configured to releasably engage the housing. The tissue collection cartridge defines a port configured to communicate with the vacuum generator such that, during at least a portion of the manual actuation of the trigger to drive the drive assembly, cut tissue is urged from the vacuum generator through the port into the tissue collection cartridge.

### BRIEF DESCRIPTION OF THE DRAWINGS

The above and other aspects and features of the present disclosure will become more apparent in light of the following detailed description when taken in conjunction with the accompanying drawings wherein like reference numerals identify similar or identical elements.
FIG 1 is a perspective view of a proximal portion of a tissue resecting instrument provided in accordance with the present disclosure;
FIGS. 2A and 2B are side views of a distal portion of an end effector assembly of the tissue resecting instrument of FIG. 1 with an inner cutting shaft of the end effector assembly disposed in more-proximal and more-distal positions, respectively;
FIGS. 3A and 3B are side views of a distal portion of another end effector assembly configured for use with the tissue resecting instrument of FIG. 1 with an inner cutting shaft of the end effector assembly disposed in first and second rotational positions, respectively;
FIG. 4 is a longitudinal, cross-sectional view of the proximal portion of the tissue resecting instrument of FIG. 1 including an adjustable resistance mechanism provided in accordance with the preset disclosure coupled to the trigger;
FIGS. 5A-5C are longitudinal, cross-sectional views of the fixed handle portion of the tissue resecting instrument of FIG. 1 including the adjustable resistance mechanism of FIG. 4 disposed in first, second, and third configurations, respectively;
FIG. 6 is a longitudinal, cross-sectional view of the proximal portion of the tissue resecting instrument of FIG. 1 including another adjustable resistance mechanism provided in accordance with the preset disclosure coupled to the trigger;
FIGS. 7A-7C are longitudinal, cross-sectional views of the fixed handle portion of the tissue resecting instrument of FIG. I including the adjustable resistance mechanism of FIG. 6 disposed in first, second, and third configurations, respectively;
FIG. 8 is a longitudinal, cross-sectional view of the proximal portion of another tissue resecting instrument provided in accordance with the preset disclosure; and
FIG. 9 is a longitudinal, cross-sectional view of the proximal portion of still another tissue resecting instrument provided in accordance with the preset disclosure.

### DETAILED DESCRIPTION

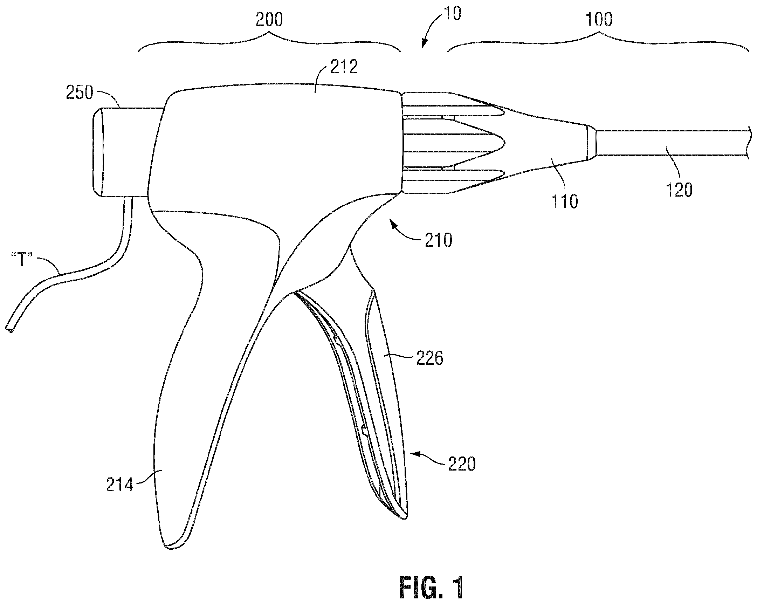

Referring generally to FIG. 1, a tissue resecting instrument 10 provided in accordance with the present disclosure configured for manual actuation to resect and remove tissue includes an end effector assembly 100 and a handpiece assembly 200. Tissue resecting instrument 10 may be adapted to connect to a fluid collection reservoir (not shown) via outflow tubing "T" for collecting fluid suctioned through tissue resecting instrument 10 during use or, alternatively, may be configured to internally retain the fluid suctioned therethrough, e.g., via internal outflow tubing and an internal fluid collection reservoir (not shown).

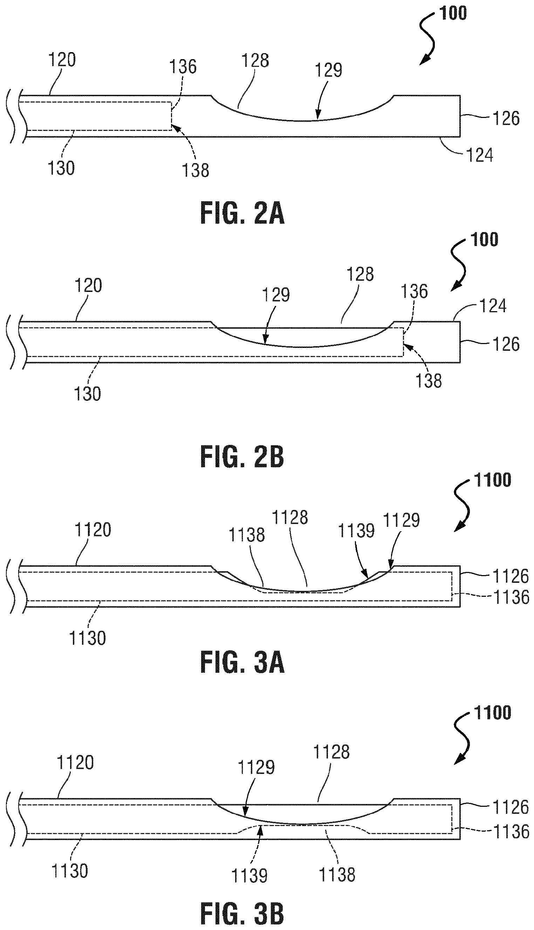

With continued reference to FIG. 1, tissue resecting instrument 10 may be configured as a single-use instrument that is discarded after use or sent to a manufacturer for reprocessing, a reusable instrument capable of being cleaned and/or sterilized for repeated use by the end-user, or a partially-single-use, partially-reusable instrument. With respect to partially-single-use, partially-reusable configurations, handpiece assembly 200 may be configured as a cleanable/sterilizable, reusable component, while end effector assembly 100 is configured as a single-use, disposable/reprocessable component, or vice versa. In any of the above configurations, end effector assembly 100 may be configured to releasably engage handpiece assembly 200 to facilitate disposal/reprocessing of any single-use components and cleaning and/or sterilization of any reusable components. Further, enabling releasable engagement of end effector assembly 100 with handpiece assembly 200 allows for use of different end effector assemblies, e.g., end effector assembly 100 (FIGS. 2A and 2B) or end effector assembly 1100 (FIGS. 3A and 3B), with handpiece assembly 200. In other embodiments, end effector assembly 100 is permanently secured to handpiece assembly 200.

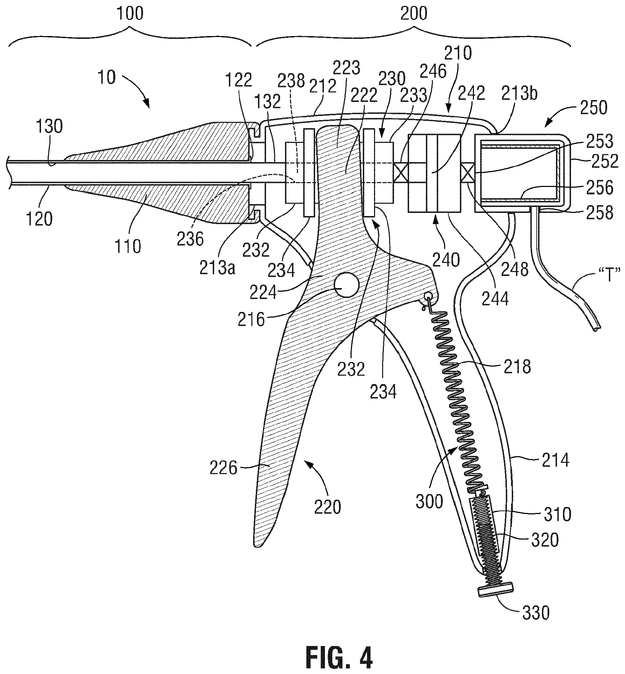

Referring to FIGS. 2A, 2B, and 4, end effector assembly 100 includes a proximal hub 1 10 which may be formed as a rotation knob configured to rotatably engage handpiece assembly 200 (or may be configured to fixedly engage handpiece assembly 200), an outer shaft 120 fixedly engaged with and extending distally from proximal hub 110, an inner cutting shaft 130 movable disposed within outer shaft 120, and an inner drive hub 140 coupled to inner cutting shaft 130 such that movement imparted to inner drive hub 140, e.g., via handpiece assembly 200, as detailed below, drives translation and, in embodiments, translation and/or rotation, of inner cutting shaft 130 within and relative to outer shaft 120.

Outer shaft 120 of end effector assembly 100, includes a proximal end portion 122 fixedly engaged with proximal hub 110. Outer shaft 120 further includes a distal end portion 124 defining a closed distal end 126 and a window 128 proximally-spaced from closed distal end 126. Window 128 provides access to the interior of outer shaft 120 and may be surrounded by a cutting edge 129 about the outer perimeter of window 128 so as to facilitate cutting of tissue passing through window 128 and into outer shaft 120.

Inner cutting shaft 130 defines a proximal end portion 132 and a distal end portion 134 defining an open distal end 136. Inner cutting shaft 130 defines an annular cutting edge 138 surrounding open distal end 136 so as to facilitate cutting of tissue passing into inner cutting shaft 130 via open distal end 136. Inner cutting shaft 130 is translatable and, in embodiments, translatable and/or rotatable, within and relative to outer shaft 120. More specifically, inner cutting shaft 130 is configured to translate distally and proximally in a reciprocating motion such that annular cutting edge 138 is exposed within window 128 of outer shaft 120 during at least a portion of the reciprocation motion of inner cutting shaft 130 to enable cutting of tissue extending through window 128. As detailed below, suction is provided to facilitate drawing tissue into window 128 and, thus, cutting and removal of tissue through inner cutting shaft 130. Inner drive hub 140 is engaged about proximal end portion 132 of inner cutting shaft 130.

With momentary reference to FIGS. 3A and 3B, another embodiment of an end effector assembly 1100 configured for use with tissue resecting instrument 10 (FIG. 1) is shown. End effector assembly 1100 is similar to and may include any of the features of end effector assembly 100 (FIGS. 2A-2B), except that, rather than providing reciprocation (and, in embodiments, rotation), inner cutting shaft 1130 of end effector assembly 1100 is longitudinally fixed and rotatable relative to outer shaft 1120. End effector assembly 1100 further differs from end effector assembly 100 (FIG. 2A) in that outer shaft 1120 and inner cutting shaft 1130 both define window 1128, 1138 proximally-spaced from the respective distal end 1126, 1136 thereof. Window 1128 and/or window 1138 may be surrounded by a cutting edge 1129, 1139, respectively, configured to facilitate cutting of tissue passing through windows 1128, 1138 upon relative rotation between windows 1128, 1138, e.g., as a result of rotation of inner cutting shaft 1130 relative to outer shaft 1120. Other suitable end effector assemblies including various different outer shaft and inner cutting shaft configurations are also contemplated.

Referring to FIGS. 1 and 4, handpiece assembly 200 generally includes a handle housing 210, a trigger 220 pivotably coupled to handle housing 210, a drive assembly 230 disposed within handle housing 210 and operably coupled to trigger 220, a vacuum generator 240 disposed within handle housing 210 and operably coupled to drive assembly 230, a tissue collection cartridge 250 releasably coupled to handle housing 210 (although in other embodiments tissue collection cartridge is permanently coupled to handle housing 210) and operably coupled to vacuum generator 240, and an adjustable resistance mechanism 300. Outflow tubing "T" couples tissue collection cartridge 250 to the fluid collection reservoir (not shown) for collecting fluid suctioned through tissue resecting instrument 10 during use. Alternatively, as noted above, handle housing 210 may include internal outflow tubing and an internal fluid collection reservoir (not shown) coupled to tissue collection cartridge 250 to retain the fluid suctioned through tissue resecting instrument 10 during use within tissue resecting instrument 10. Further, as also noted above, tissue resecting instrument 10 may be configured for powered actuation and, in such embodiments, a motor and one or more actuation buttons (not shown) replaces trigger 220. Other suitable powered or manual actuators are also contemplated.

Handle housing 210 defines a pistol-grip configuration, although other configurations are also contemplated, and includes a barrel portion 212 and a fixed handle portion 214 depending from barrel portion 212. Barrel portion 212 includes a distal port 213a about which proximal hub 110 of end effector assembly 100 is configured to releasably engage handle housing 210, e.g., via snap-fit engagement, with inner cutting shaft 130 and inner drive hub 140 extending through distal port 213a into handle housing 210. Barrel portion 212 also includes a proximal port 213b configured to receive (releasably or permanently) at least a portion of tissue collection cartridge 250, e.g., in threaded engagement, friction-fit engagement, etc. Barrel portion 212 of handle housing 210 houses drive assembly 230 and vacuum generator 240 therein.

Handle housing 210 supports a pivot 216 therein about which trigger 220 is pivotably coupled, thereby enabling trigger 220 to pivot relative to fixed handle portion 214 of handle housing 210 through an actuation stroke between an un-actuated position, wherein trigger 220 is further-spaced from fixed handle portion 214, and an actuated position, wherein trigger 220 is closer to fixed handle portion 214. The actuation stroke of trigger 220 includes a forward stroke portion involving movement of trigger 220 from the un-actuated position to the actuated position, and a return stroke portion, involving movement of trigger 220 from the actuated position back to the un-actuated position. Fixed handle portion 214 further supports adjustable resistance mechanism 300. Adjustable resistance mechanism 300, as detailed below, is coupled between fixed handle portion 214 and trigger 220 to bias trigger 220 towards the un-actuated position and to provide an adjustable resistance to pivoting of trigger 220 against the bias from the un-actuated position towards the actuated position.

Trigger 220 includes an upper drive portion 222, an intermediate pivot portion 224, and a lower manipulation portion 226, and may be formed as a single, monolithic piece or may otherwise be formed as a unitary structure . Intermediate pivot portion 224 of trigger 220 is pivotably coupled to handle housing 210 about pivot 216. Upper drive portion 222 of trigger 220 extends upwardly from intermediate pivot portion 224 into handle housing 210 to operably couple to drive assembly 230. More specifically, upper drive portion 222 defines a bifurcated configuration including a pair of spaced-apart upper drive flanges 223 disposed on either side of drive assembly 230 and operably coupled thereto. Lower manipulation portion 226 of trigger 220 extends downwardly from intermediate pivot portion 224 exteriorly from handle housing 210 to enable manual manipulation thereof by a user between the un-actuated and actuated positions.

Drive assembly 230 of handpiece assembly 200 includes a mandrel 232 engaged about an inner shaft 236 defining a lumen 238 extending therethrough. Mandrel 232 includes a body 233 and pair of longitudinally-spaced rims 234 disposed on body 233 and is configured to receive upper drive flanges 223 of upper drive portion 222 of trigger 220 longitudinally between rims 234 on either side of body 233. In this manner, pivoting of trigger 220 towards the actuated position urges upper drive flanges 223 into the distal rim of the pair of longitudinally-spaced rims 234 to thereby urge mandrel 232 distally through handle housing 210. On the other hand, pivoting of trigger 220 towards the un-actuated position urges drive flanges 223 into the proximal rim of the pair of longitudinally-spaced rims 234 to thereby urge mandrel 232 proximally through handle housing 210.

Inner drive hub 140 of end effector assembly 100 is configured to releasably engage inner shaft 236 and/or mandrel 232, e.g., via mechanical fastening, friction-fit engagement, magnetic coupling, etc., upon engagement of end effector assembly 100 with handpiece assembly 100 such that inner drive hub 140 is fixed relative to mandrel 232, and such that the interior of inner cutting shaft 130 of end effector assembly 100 is disposed in fluid communication with lumen 238 of inner shaft 236. Of course, in embodiments where end effector assembly 100 is permanently secured to handpiece assembly 200, inner drive hub 140 of end effector assembly 100 is permanently engaged to mandrel 232.

With inner drive hub 140 fixed relative to mandrel 232, translation of mandrel 232, e.g., in response to pivoting of trigger 220, likewise translates inner cutting shaft 130 through and relative to outer shaft 120, More specifically, pivoting of trigger 220 relative to handle housing 210 between the un-actuated position and the actuated position translates inner cutting shaft 130 through and relative to outer shaft 120 between a more-proximal position (FIG. 2A) and a more-distal position (FIG. 2B).

With momentary additional reference to FIGS. 3A and 3B, in embodiments where the end effector assembly, e.g., end effector assembly 1100, includes an inner cutting shaft 1130 configured to rotate relative to the outer shaft 1120, rather than inner drive hub 140 being fixed relative to mandrel 232, mandrel 232 may include a coupler (not shown) that is operably engaged within a helical channel (not shown) defined on or otherwise associated with inner drive hub 140. As a result of such a configuration, longitudinal translation of mandrel 232, e.g., in response to actuation of trigger 220, effects rotation of inner drive hub 140 and, thus, inner cutting shaft 1130.

In embodiments where the end effector assembly, e.g., end effector assembly 1100, includes an inner cutting shaft 1130 configured to both reciprocate and rotate relative to the outer shaft 1120, rather than inner drive hub 140 being fixed relative to mandrel 232, mandrel 232 or inner drive hub 140 may include a helical channel (not shown) defined thereon or otherwise associated therewith and a coupler (not shown) may be fixed within handle housing 210 and engaged within the helical channel. As a result of such a configuration, longitudinal translation of mandrel 232, e.g., in response to actuation of trigger 220, effects both rotation and translation of inner drive hub 140 and, thus, inner cutting shaft 1130.

Referring again to FIGS. 1 and 4, vacuum generator 240 is disposed within handle housing 210 and operably coupled to drive assembly 230. Vacuum generator 240 includes a chamber 244 that is disposed in fluid communication with lumen 238 of inner shaft 236 of drive assembly 230 which, as noted above, is disposed in fluid communication with the interior of inner cutting shaft 130 of end effector assembly 100. As a result, vacuum generated by vacuum generator 240 suctions tissue and fluid through window 128 of outer shaft 120, open distal end 136 of inner cutting shaft 130, lumen 238 of inner shaft 236 of drive assembly 230, and into chamber 244 of vacuum generator 240,

Vacuum generator 240, more specifically, includes a plunger 242 sealingly engaged and slidably disposed within chamber 244. In embodiments, inner shaft 236 defines the push-rod of plunger 242, although other configurations are also contemplated. Plunger 242 is coupled with mandrel 232, e.g., via inner shaft 236 or in any other suitable manner, such that as mandrel 232 is translated through handle housing 210, plunger 242 is similarly translated through chamber 244. More specifically, when mandrel 232 is translated distally, e.g., in response to movement of trigger 220 from the un-actuated position towards the actuated position to move inner cutting shaft 130 from the more-proximal position (FIG. 2A) towards the more-distal position (FIG. 2B), plunger 242 is moved distally through chamber 244 to increase the volume of chamber 244 and generate vacuum within chamber 244, thereby establishing suction through lumen 238 of inner shaft 236 and inner cutting shaft 130. In this manner, as inner cutting shaft 130 is moved from the more-proximal position (FIG. 2A) towards the more-distal position (FIG. 2B), tissue and fluid are suctioned through window 128 of outer shaft 120, tissue is cut by open distal end 136 of inner cutting shaft 130, and the cut tissue and fluid are suctioned proximally through lumen 238 of inner shaft 236 of drive assembly 230 and into chamber 244 of vacuum generator 240.

When mandrel 232 is returned proximally, e.g., in response to movement of trigger 220 from the actuated position back towards the un-actuated position to move inner cutting shaft 130 from the more-distal position (FIG. 2B) back towards the more-proximal position (FIG. 2A), plunger 242 is moved proximally through chamber 244 to push tissue and fluid, under pressure, from chamber 244 of vacuum generator 240 into tissue collection cartridge 250. One-way valves 246, 248 are disposed between vacuum generator 240 and drive assembly 230 and between vacuum generator 240 and tissue collection cartridge 250, respectively, to inhibit pumping tissue and fluid distally from vacuum generator 240 into lumen 238 of inner shaft 236 and drawing tissue and fluid distally from tissue collection cartridge 250 back into vacuum generator 240, respectively.

Tissue collection cartridge 250, as noted above, is releasably coupled to handle housing 210. Tissue collection cartridge 250, more specifically, may be configured to releasably engage proximal port 213b of handle housing 210 via threaded engagement or other suitable engagement. Tissue collection cartridge 250 includes an outer housing 252 defining a distal port 253 configured to couple, in fluid communication, with chamber 244 of vacuum generator 240 upon engagement of tissue collection cartridge 250 with handle housing 210, In this manner, tissue and fluid suctioned through window 128 of outer shaft 120, open distal end 136 of inner cutting shaft 130, lumen 238 of inner shaft 236 of drive assembly 230, and into chamber 244 of vacuum generator 240, may then be urged into tissue collection cartridge 250. One-way valve 248, as an alternative to being part of vacuum generator 240, may be disposed within distal port 253 of tissue collection cartridge 250.

Tissue collection cartridge 250 further includes an internal filter 256 disposed within outer housing 252 that is configured to permit passage of fluid therethrough but inhibit the passage of tissue therethrough. An outflow port 258 configured to enable connection of outflow tubing "T" with tissue collection cartridge 250 enables the fluid that passes through filter 256 to dram out from tissue collection cartridge 250 to a fluid collection reservoir (not shown).

Referring generally to FIGS. 1-2B and 4, in preparation for use, if not already done so, end effector assembly 100 is engaged with handpiece assembly 200, tissue collection cartridge 250 is engaged with handle housing 210 of handpiece assembly 200, and outflow tubing "T" is coupled between tissue collection cartridge 250 and the fluid collection reservoir (not shown). In embodiments, any or all of the above engagements and/or couplings are accomplished during manufacturing and, thus, need not be performed by the end-user.

With tissue resecting instrument 10 assembled as detailed above, in use, tissue resecting instrument 10 is inserted into an internal body cavity or organ, e.g., a uterus, such that the distal end portion of end effector assembly 100 is positioned adjacent tissue to be removed. Tissue resecting instrument 10 may be inserted through an endoscope, e.g., a hysteroscope, or other instrument, or may be used independently. Once tissue resecting instrument 10 is positioned as desired adjacent tissue to be removed, tissue resecting instrument 10 is activated by pivoting trigger 220 relative to fixed handle portion 214 of handle housing 210 through the actuation stroke from the un-actuated position to the actuated position and back to the un-actuated position to thereby reciprocate inner cutting shaft 130 through and relative to outer shaft 120 (e.g., from the more-proximal position (FIG. 2A) to the more-distal position (FIG. 2B) and back to the more-proximal position (FIG. 2A)), suction cut tissue and fluid through inner cutting shaft 130 and lumen 238 of inner shaft 236 into vacuum generator 240, and urge tissue and fluid from vacuum generator 240 into tissue collection cartridge 250. Tissue resecting instrument 10 may be repeatedly actuated as detailed above to cut and remove target tissue as desired. The tissue urged into tissue collection cartridge 250 during use is retained therein, while the fluid urged into tissue collection cartridge 250 passes through filter 256, outflow port 258, and outflow tubing "T" to the fluid collection reservoir (not shown).

Once the desired tissue is removed, tissue resecting instrument 10 may be removed from the surgical site. Thereafter, end effector assembly 100 and tissue collection cartridge 250 may be disengaged from handpiece assembly 200. End effector assembly 100 and/or handpiece assembly 200 may then be discarded, sent for reprocessing, or sterilized for reuse. Tissue collection cartridge 250 may be sent to pathology for analyzing the tissue retained therein or may likewise be discarded.

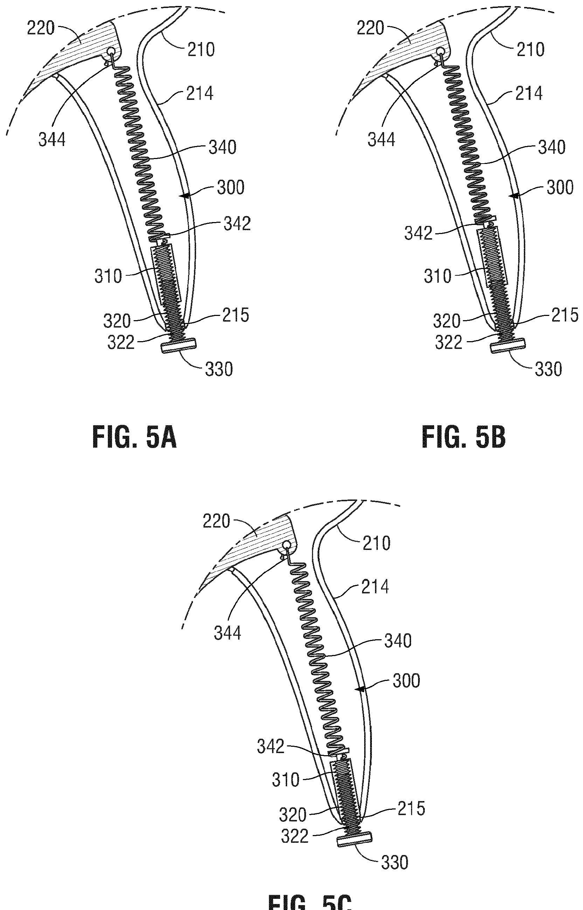

Referring to FIG. 4, in conjunction with FIGS. 5A-5C, adjustable resistance mechanism 300, as noted above, is coupled between fixed handle portion 214 and trigger 220 to bias trigger 220 towards the un-actuated position and to provide an adjustable resistance to pivoting of trigger 220 against the bias from the un-actuated position towards the actuated position. For example, a user may desire a lower resistance to pivoting trigger 220 from the un-actuated position towards the actuated position to ease actuation force and reduce fatigue and, thus, may adjust the adjustable resistance mechanism 300 from a first configuration (FIG. 5A) to a second configuration (FIG. 5B) that provides a smaller resistance than the first configuration. On the other hand, as another example, a user may desire a greater resistance to pivoting trigger 220 from the un-actuated position towards the actuated position to enable greater control and "feel" and, thus, may adjust the adjustable resistance mechanism 300 from the first configuration (FIG. 5A) to a third configuration (FIG. 5C) that provides a greater resistance than the first configuration.

With reference to FIGS. 5A-5C, adjustable resistance mechanism 300 includes an internally threaded shaft 310, an externally threaded shaft 320, a control knob 330, and a spring 340, e.g., a coil spring. Internally threaded shaft 310 is disposed within fixed handle portion 214 of handle housing 210 and is movable therethrough. Externally threaded shaft 320 is threadingly engaged within internally threaded shaft 310 and is rotatable but otherwise fixed relative to fixed handle portion 214 of handle housing 210. More specifically, a neck 322 of externally threaded shaft 320 may be rotatably captured within a socket 215 defined within fixed handle portion 214. In this manner, when externally threaded shaft 320 is rotated relative to fixed handle portion 214, internally threaded shaft 310 is translated through and relative to fixed handle portion 214. Control knob 330 extends from neck 322 externally of fixed handle portion 214 to enable manual manipulation, e.g., rotation, thereof by a user to adjust the resistance provided by adjustable resistance mechanism 300. That is, rotation of control knob 330 relative to fixed handle portion 214 rotates externally threaded shaft 320 relative to fixed handle portion 214 to, as noted above, translate internally threaded shaft 310 through and relative to fixed handle portion 214. In embodiments, externally threaded shaft 320 and control knob 330 are monolithically or otherwise formed as a unitary component.

Spring 340 is fixed, e.g., wrapped around and/or through, relative to internally threaded shaft 310 at a first end portion 342 thereof and is fixed, e.g., wrapped around and/or through, trigger 220 at a second end portion 344. Second end portion 344 of spring 340 is fixed relative to trigger 220 on an opposite side of pivot 216 as compared to lower manipulation portion 226 (see FIG. 4) such that, as noted above, spring 340 provides a resistance to pivoting of trigger 220 from the un-actuated position towards the actuated position.

As detailed above, rotation of control knob 330 relative to fixed handle portion 214 rotates externally threaded shaft 320 relative to fixed handle portion 214 to, as also detailed above, translate internally threaded shaft 310 through and relative to fixed handle portion 214. More specifically, with second end portion 344 of spring 340 engaged with internally threaded shaft 310, rotation of control knob 330 in a first direction urges internally threaded shaft 310 towards an extended, less-overlapping position relative to externally threaded shaft 320 to transition adjustable resistance mechanism 300 from a first configuration (FIG. 5A), wherein spring 340 defines a first initial length and, thus, provides a first initial resistance to actuation, to a second configuration (FIG. 5B), wherein spring 340 defines a second initial length shorter than the first initial length and, thus, provides a second initial resistance to actuation that is less than the first initial resistance. On the other hand, rotation of control knob 330 in a second, opposite direction urges internally threaded shaft 310 towards a retracted, more-overlapping position relative to externally threaded shaft 320 to transition adjustable resistance mechanism 300 from the first configuration (FIG. 5A), wherein spring 340 defines the first initial length and, thus, provides the first initial resistance to actuation, to a third configuration (FIG. 5C), wherein spring 340 defines a third initial length longer than the first initial length and, thus, provides a third initial resistance to actuation that is greater than the first initial resistance. As an alternative or in addition to providing first, second, and third initial resistances in the first, second, and third configurations, respectively, spring 340 may provide first, second, and third different resistance profiles, e.g., wherein the spring 340 provides a variable resistance throughout compression/extension thereof such as with respect to a coil spring according to Hooke's law, in the first, second, and third configurations, respectively. The second and third resistance profiles may be less and greater, respectively, than the first resistance profile on average and/or at each point during actuation over a portion or the entire forward stroke of trigger 220.

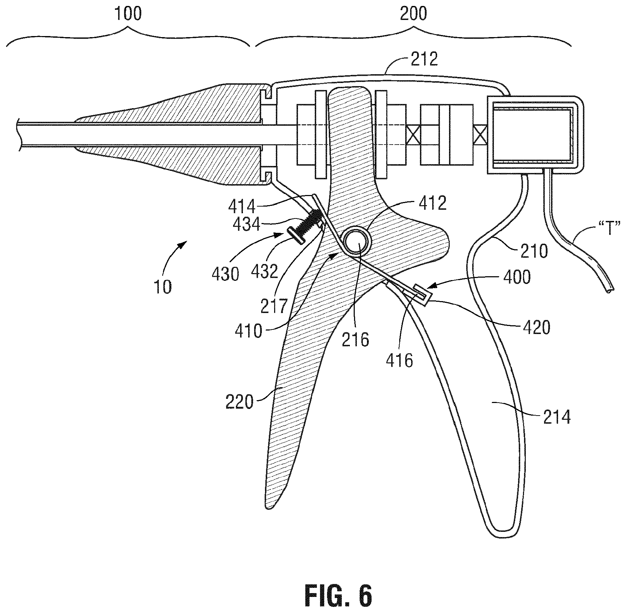

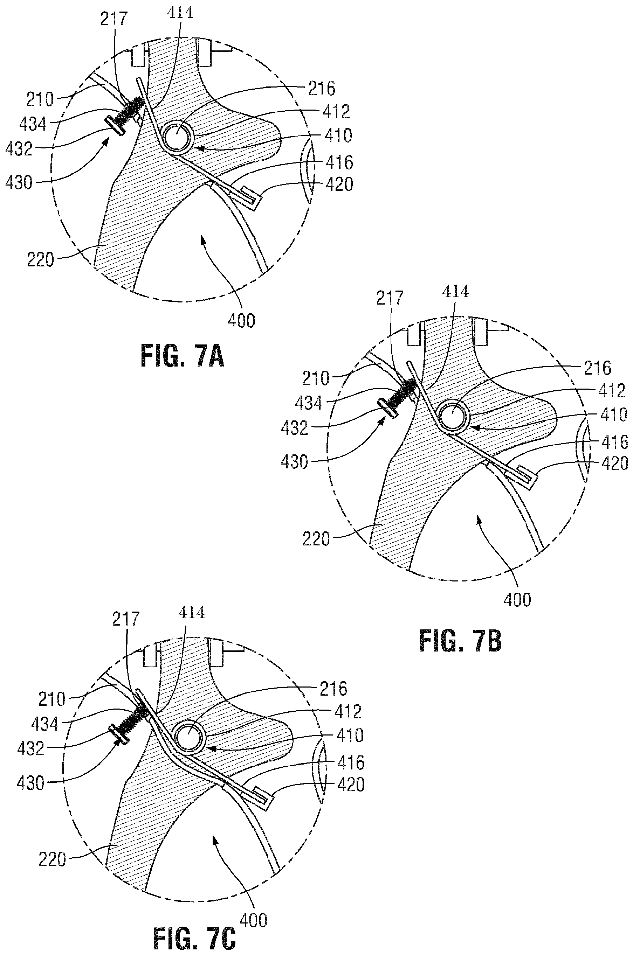

Referring to FIG. 6, in conjunction with FIGS. 7A-7C, another adjustable resistance mechanism 400 configured for use with handpiece assembly 200 of tissue resecting instrument 10 is shown. Adjustable resistance mechanism 400 is coupled about pivot 216 and between handle housing 210 and trigger 220 to bias trigger 220 towards the un-actuated position and to provide an adjustable resistance to pivoting of trigger 220 against the bias from the un-actuated position towards the actuated position. Similarly as detailed above with respect to adjustable resistance mechanism 300 (FIGS. 4 and 5A-5C), a user may desire a lower resistance to pivoting trigger 220 from the un-actuated position towards the actuated position and, thus, may adjust the adjustable resistance mechanism 400 from a first configuration (FIG. 7A) to a second configuration (FIG. 7B) that provides a smaller resistance than the first configuration. On the other hand, a user may desire a greater resistance to pivoting trigger 220 from the un-actuated position towards the actuated position and, thus, may adjust the adjustable resistance mechanism 400 from the first configuration (FIG. 7A) to a third configuration (FIG. 7C) that provides a greater resistance than the first configuration.

With reference to FIGS. 7A-7C, adjustable resistance mechanism 400 includes a torsion spring 410, a capture block 420, and a control member 430 including a control knob 432 and a control shaft 434. Torsion spring 410 includes a body 412 that is disposed about pivot 216 and operably coupled with trigger 220 (via direct or indirect coupling) and first and second legs 414, 416 extending from body 412. First leg 414 is operably positioned relative to control shaft 434 of control member 430, as detailed below. Second leg 416 is captured within capture block 420 which is fixed (and in some embodiments, formed integrally) within handle housing 210 such that second leg 416 is fixed relative to handle housing 210.

Control member 430, as noted above, includes control knob 432 and control shaft 434 which, in embodiments, may be monolithically or otherwise formed as a unitary component. Control shaft 434 defines external threading and is threadingly engaged within a threaded socket 217 defined within handle housing 210. Control knob 432 is externally disposed of handle housing 210 to enable manual manipulation, e.g., rotation, thereof. More specifically, rotation of control knob 432 relative to handle housing 210 drives control shaft 434 further into handle housing 210 or backs control shaft 434 further out of handle housing 210, depending upon the direction of rotation.

First leg 414 of torsion spring 410, as noted above, is operably positioned relative to control shaft 434 of control member 430 while second leg 416 of torsion spring 410, as also noted above, is captured within capture block 420 and fixed relative to handle housing 210. More specifically, first leg 414 is positioned such that control shaft 434 abuts a side surface thereof. Thus, rotation of control knob 432 to drive control shaft 434 further into handle housing 210 urges control shaft 434 against first leg 414 to thereby move first leg 414 to rotate in a first direction about body 412 and relative to second leg 416, increasing the initial tension on torsion spring 410. On the other hand, rotation of control knob 432 to back control shaft 434 further out of handle housing 210 pulls control shaft 434 away from first leg 414 to enable first leg to follow control shaft 434, under the bias of torsion spring 410, decreasing the initial tension on torsion spring 410.

Referring to FIG. 7A, in a first configuration of adjustable resistance mechanism 400, control shaft 434 retains first leg 414 of torsion spring 410 in a first position corresponding to a first initial tension on torsion spring 410 and, thus, provides a first initial resistance to actuation of trigger 220. With additional reference to FIG. 7B, rotating control knob 432 in a first direction urges first leg 414 of torsion spring 410 to a second position corresponding to a second initial tension on torsion spring 410 greater than the first initial tension and, thus, provides a second initial resistance to actuation of trigger 220 that is greater than the first initial resistance. As such, adjustable resistance mechanism 400 is transitioned from the first configuration (FIG. 7A) to a second configuration (FIG. 7B).

As shown in FIGS. 7A and 7C, rotating control knob 432 in a second direction urges first leg 414 of torsion spring 410 to a third position corresponding to a third initial tension on torsion spring 410 smaller than the first initial tension and, thus, provides a third initial resistance to actuation of trigger 220 that is less than the first initial resistance. As such, adjustable resistance mechanism 400 is transitioned from the first configuration (FIG. 7A) to a third configuration (FIG. 7B).

As an alternative or in addition to providing first, second, and third initial resistances in the first, second, and third configurations, respectively, torsion spring 410 may provide second and third different resistance profiles that may be greater and less, respectively, than the first resistance profile on average and/or at each point during actuation over a portion or the entire forward stroke of trigger 220.

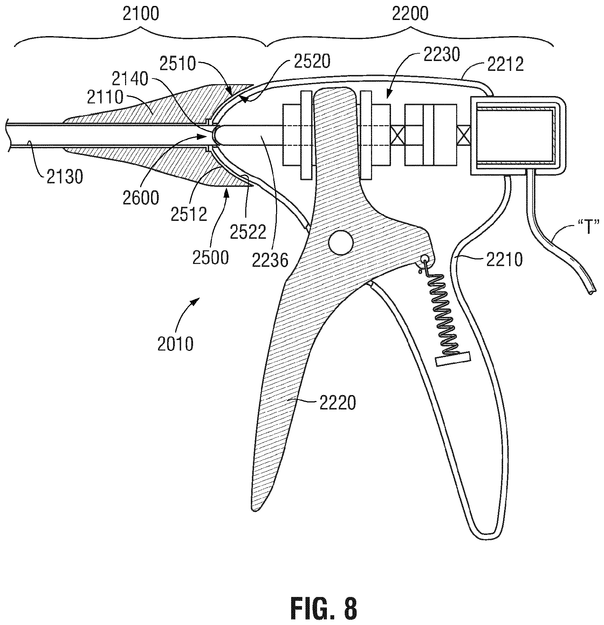

Turning to FIG. 8, another tissue resecting instrument 2010 provided in accordance with the present disclosure for manual actuation to resect and remove tissue includes an end effector assembly 2100 and a handpiece assembly 2200. Tissue resecting instrument 2010 may be adapted to connect to a fluid collection reservoir (not shown) via outflow tubing "T" for collecting fluid suctioned through tissue resecting instrument 2010 during use or, alternatively, may be configured to internally retain the fluid suctioned therethrough, e.g., via internal outflow tubing and an internal fluid collection reservoir (not shown). Tissue resecting instrument 2010 is similar to and may include any of the features of tissue resecting instrument 10 (FIGS. 1-2B and 4) except as specifically contradicted below and, thus, similarities therebetween are summarily described below or omitted entirely in order to avoid unnecessary repetition. Tissue resecting instrument 2010, for example, may include an adjustable resistance mechanism 300 (FIG. 4), 400 (FIG. 6), or such may be omitted.

Tissue resecting instrument 2010 includes an isolation mechanism 2500 that couples proximal hub 2110 of end effector assembly 2100 with barrel portion 2212 of handle housing 2210 of handpiece assembly 2200. Isolation mechanism 2500 includes a ball and socket joint configuration wherein one of a proximal portion of proximal hub 2110 of end effector assembly 2100 or a distal portion of barrel portion 2212 of handle housing 2210 defines a concave receiving surface 2510 defining a socket 2512 and the other of the proximal portion of proximal hub 2110 of end effector assembly 2100 or the distal portion of barrel portion 2212 of handle housing 2210 defines a convex protruding surface 2520 defining a ball 2522 configured for receipt within socket 2512. Ball 2522 may be captured within socket 2512 or otherwise coupled thereto to maintain engagement therebetween. With ball 2522 received within socket 2512, handle housing 2210 may yaw and/or pitch within a range of motion relative to proximal hub 2110 and, thus, end effector assembly 2100, without effecting movement, or dampening movement, of end effector assembly 2100. In this manner, isolation mechanism 2500 isolates handle housing 2210 from end effector assembly 2100 over a range of motion to inhibit inadvertent movements of handle housing 2210 from effecting the position of end effector assembly 2100. Such a configuration is advantageous, for example, such that end effector assembly 2100 remains stationary or movement thereof is dampened when the user is actuating trigger 2220 (and inadvertently moves handle housing 2210 while actuation trigger 2200). Movement of handle housing 2210 outside of the range of motion, on the other hand, results in corresponding movement of end effector assembly 2100, thus permitting manual manipulation thereof when desired.

A locking mechanism (not shown) may be provided to selectively lock proximal hub 2110 relative to barrel portion 2212 of handle housing 2210, thus effectively disabling isolation mechanism 2500 when use thereof is not desired.

In embodiments, inner drive hub 2140 of end effector assembly 2100 is coupled with inner shaft 2236 of drive assembly 2230 via a connection mechanism 2600, e.g., a ball and socket joint, a flexible tube, a bellows, etc., that enables isolation of motion of handle housing 2210 relative to end effector assembly 2100, as detailed above, while permitting actuation of drive assembly 2230 to rotate and/or translate inner cutting shaft 2130 in each of an aligned, yawed, and/or pitched orientation of handle housing 2210 relative to end effector assembly 2100.

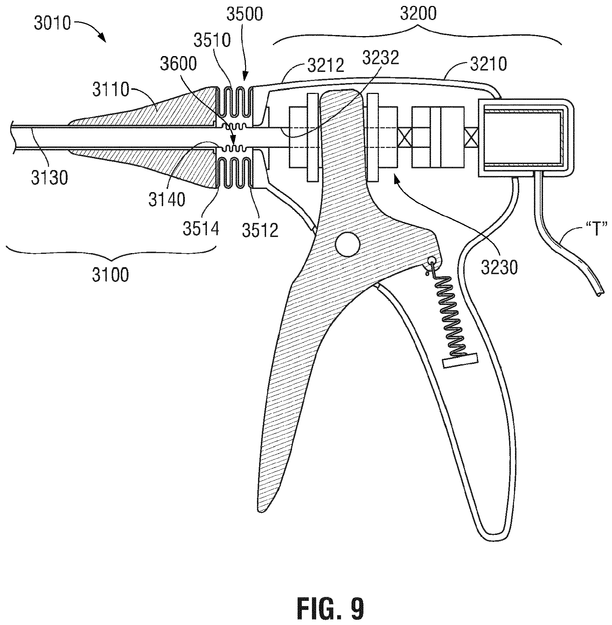

Turning to FIG. 9, another tissue resecting instrument 3010 is provided in accordance with the present disclosure. Except as specifically contradicted below, tissue resecting instrument 3010 is similar to and may include any of the features of tissue resecting instruments 10 (FIGS. 1-2B and 4), 3010 (FIG. 8).

Tissue resecting instrument 3010 includes an isolation mechanism 3500 that couples proximal hub 3110 of end effector assembly 3100 with barrel portion 3212 of handle housing 3210 of handpiece assembly 3200. Isolation mechanism 3500 includes a bellows 3510 engaged at a proximal end portion 3512 to barrel portion 3212 and at a distal end portion 3514 to proximal hub 3110, thus enabling handle housing 3210 to yaw and/or pitch within a range of motion relative to proximal hub 3110 and, thus, end effector assembly 3100, without effecting movement, or dampening movement, of end effector assembly 3100. In this manner, isolation mechanism 3500 isolates handle housing 3210 from end effector assembly 3100 over a range of motion to inhibit inadvertent movements of handle housing 3210 from effecting the position of end effector assembly 3100. A locking mechanism (not shown) or a rigidizing mechanism (not shown) may be provided to selectively lock proximal hub 3110 relative to barrel portion 3212 of handle housing 3210 or rigidize bellows 3510, respectively, thus effectively disabling isolation mechanism 3500 when use thereof is not desired.

In embodiments, inner drive hub 3140 of end effector assembly 3100 is coupled with inner shaft 3236 of drive assembly 3230 via a connection mechanism 3600, e.g., a ball and socket joint, a flexible tube, a bellows, etc., that enables isolation of motion of handle housing 3210 relative to end effector assembly 3100, as detailed above, while permitting actuation of drive assembly 3230 to rotate and/or translate inner cutting shaft 3130 in each of an aligned, yawed, and/or pitched orientation of handle housing 3210 relative to end effector assembly 3100.

From the foregoing and with reference to the various drawings, those skilled in the art will appreciate that certain modifications can be made to the present disclosure without departing from the scope of the same. While several embodiments of the disclosure have been shown in the drawings, it is not intended that the disclosure be limited thereto, as it is intended that the disclosure be as broad in scope as the art will allow and that the specification be read likewise. Therefore, the above description should not be construed as limiting, but merely as exemplifications of particular embodiments. Those skilled in the art will envision other modifications within the scope and spirit of the claims appended hereto.

### The invention may be described by reference to the following numbered paragraphs:-

1. A tissue resecting instrument, comprising:
   a housing;
   an outer shaft extending distally from the housing and defining a window at a distal end potion thereof;
   an inner cutting shaft extending through the outer shaft, the inner cutting shaft at least one of translatable or rotatable relative to the outer shaft to cut tissue extending through the window;
   a drive assembly coupled to the inner cutting shaft and configured to drive the at least one of translation or rotation of the inner cutting shaft;
   a trigger coupled to the drive assembly, wherein manual actuation of the trigger actuates the drive assembly to drive the at least one of translation or rotation of the inner cutting shaft; and
   an adjustable resistance mechanism including a spring coupled between the trigger and the housing, and a control knob operably coupled to the spring to enable adjustment of a resistance to pivoting of the trigger from an un-actuated position to an actuated position.
2. The tissue resecting instrument according to paragraph 1, wherein the spring is a coil spring and wherein adjustment of the resistance to pivoting includes adjusting an initial length of the coil spring.
3. The tissue resecting instrument according to paragraph 2, wherein the coil spring is coupled at a first end portion thereof to the trigger and wherein a second end portion of the coil spring is movable to adjust the initial length of the coil spring.
4. The tissue resecting instrument according to paragraph 1, wherein the spring is a torsion spring and wherein adjustment of the resistance to pivoting includes adjusting a relative positioning of first and second legs of the torsion spring.
5. The tissue resecting instrument according to paragraph 4, wherein the second leg is fixed relative to the housing and wherein the first leg is rotatable about a body of the torsion spring and relative to the second leg to adjust the relative positioning of the first and second legs.
6. The tissue resecting instrument according to paragraph 1, wherein adjustment of the resistance to pivoting adjusts an initial resistance to the pivoting of the trigger from the un-actuated position to the actuated position.
7. The tissue resecting instrument according to paragraph 1, wherein adjustment of the resistance to pivoting adjusts a resistance profile through at least a portion of the pivoting of the trigger from the un-actuated position to the actuated position.
8. The tissue resecting instrument according to paragraph 1, further comprising a vacuum generator coupled to the drive assembly and the inner cutting shaft such that, during at least a portion of the manual actuation of the trigger to drive the drive assembly, the vacuum generator generates vacuum to suction cut tissue through the inner cutting shaft and into the vacuum generator.
9. The tissue resecting instrument according to paragraph 8, further comprising a tissue collection cartridge configured to releasably engage the housing, the tissue collection cartridge defining a port configured to communicate with the vacuum generator such that, during at least a portion of the manual actuation of the trigger to drive the drive assembly, cut tissue is urged from the vacuum generator through the port into the tissue collection cartridge.
10. The tissue resecting instrument according to paragraph 1, wherein the trigger is pivotably coupled to the housing and movable relative thereto between the un-actuated position and the actuated position to drive the drive assembly to translate the inner cutting shaft between a more-proximal position and a more-distal position.
11. The tissue resecting instrument according to paragraph 1, wherein the spring is further configured to bias the trigger towards the un-actuated position.
12. A tissue resecting instrument, comprising:
   an end effector assembly, including:
      a proximal hub;
      an outer shaft extending distally from the proximal hub and defining a window at a distal end potion thereof; and
      an inner cutting shaft extending through the outer shaft, the inner cutting shaft at least one of translatable or rotatable relative to the outer shaft to cut tissue extending through the window;
   a handpiece assembly, including:
      a housing;
      a drive assembly disposed within the housing and coupled to the inner cutting shaft, the drive assembly configured to drive the at least one of translation or rotation of the inner cutting shaft; and
      a trigger coupled to the drive assembly, wherein manual actuation of the trigger actuates the drive assembly to drive the at least one of translation or rotation of the inner cutting shaft; and
   an isolation mechanism operably coupled between the proximal hub and the housing to engage the proximal hub and the housing with one another, wherein the isolation mechanism isolates movement of the housing from the end effector assembly through a range of motion.
13. The tissue resecting instrument according to paragraph 12, wherein the isolation mechanism includes a ball and socket joint.
14. The tissue resecting instrument according to paragraph 13 wherein one of the proximal hub or the housing includes a concave surface defining a socket of the ball and socket joint, and wherein the other of the proximal hub or the housing includes a convex surface defining a ball of the ball and socket joint, the ball received within the socket.
15. The tissue resecting instrument according to paragraph 12, wherein the isolation mechanism includes a bellows.
16. The tissue resecting instrument according to paragraph 15 wherein a proximal end portion of the bellows is coupled to the housing and wherein a distal end portion of the bellows is coupled to the proximal hub.
17. The tissue resecting instrument according to paragraph 12, further comprising a connector operably coupling the inner cutting shaft and the drive assembly with one another.
18. The tissue resecting instrument according to paragraph 17, wherein the connector is configured to enable the drive assembly to drive the at least one of translation or rotation of the inner cutting shaft in each of an aligned position, a yawed position, and a pitched position of the housing relative to the proximal hub.
19. The tissue resecting instrument according to paragraph 12, wherein the handpiece assembly further includes a vacuum generator coupled to the drive assembly and the inner cutting shaft such that, during at least a portion of the manual actuation of the trigger to drive the drive assembly, the vacuum generator generates vacuum to suction cut tissue through the inner cutting shaft and into the vacuum generator.
20. The tissue resecting instrument according to paragraph 19, wherein the handpiece assembly further includes a tissue collection cartridge configured to releasably engage the housing, the tissue collection cartridge defining a port configured to communicate with the vacuum generator such that, during at least a portion of the manual actuation of the trigger to drive the drive assembly, cut tissue is urged from the vacuum generator through the port into the tissue collection cartridge.

Further described herein is the subject-matter of the following clauses:
1. A tissue resecting instrument, comprising:
   a housing;
   an outer shaft extending distally from the housing and defining a window at a distal end potion thereof;
   an inner cutting shaft extending through the outer shaft, the inner cutting shaft at least one of translatable or rotatable relative to the outer shaft to cut tissue extending through the window;
   a drive assembly coupled to the inner cutting shaft and configured to drive the at least one of translation or rotation of the inner cutting shaft;
   a trigger coupled to the drive assembly, wherein manual actuation of the trigger actuates the drive assembly to drive the at least one of translation or rotation of the inner cutting shaft; and
   an adjustable resistance mechanism including a spring coupled between the trigger and the housing, and a control knob operably coupled to the spring to enable adjustment of a resistance to pivoting of the trigger from an un-actuated position to an actuated position.
2. The tissue resecting instrument according to clause 1, wherein the spring is a coil spring and wherein adjustment of the resistance to pivoting includes adjusting an initial length of the coil spring.
3. The tissue resecting instrument according to clause 2, wherein the coil spring is coupled at a first end portion thereof to the trigger and wherein a second end portion of the coil spring is movable to adjust the initial length of the coil spring.
4. The tissue resecting instrument according to clause 1, wherein the spring is a torsion spring and wherein adjustment of the resistance to pivoting includes adjusting a relative positioning of first and second legs of the torsion spring.
5. The tissue resecting instrument according to clause 4, wherein the second leg is fixed relative to the housing and wherein the first leg is rotatable about a body of the torsion spring and relative to the second leg to adjust the relative positioning of the first and second legs.
6. The tissue resecting instrument according to any one of the preceding clauses, wherein adjustment of the resistance to pivoting adjusts at least one of: an initial resistance to the pivoting of the trigger from the un-actuated position to the actuated position; or a resistance profile through at least a portion of the pivoting of the trigger from the un-actuated position to the actuated position,
7. The tissue resecting instrument according to any one of the preceding clauses, further comprising a vacuum generator coupled to the drive assembly and the inner cutting shaft such that, during at least a portion of the manual actuation of the trigger to drive the drive assembly, the vacuum generator generates vacuum to suction cut tissue through the inner cutting shaft and into the vacuum generator.
8. The tissue resecting instrument according to clause 7, further comprising a tissue collection cartridge configured to releasably engage the housing, the tissue collection cartridge defining a port configured to communicate with the vacuum generator such that, during at least a portion of the manual actuation of the trigger to drive the drive assembly, cut tissue is urged from the vacuum generator through the port into the tissue collection cartridge.
9. The tissue resecting instrument according to any one of the preceding clauses, wherein the trigger is pivotably coupled to the housing and movable relative thereto between the un-actuated position and the actuated position to drive the drive assembly to translate the inner cutting shaft between a more-proximal position and a more-distal position.
10. The tissue resecting instrument according to any one of the preceding clauses, wherein the spring is further configured to bias the trigger towards the un-actuated position.

## Claims

1. A tissue resecting instrument, comprising:
an end effector assembly, including:
a proximal hub;
an outer shaft extending distally from the proximal hub and defining a window at a distal end potion thereof; and
an inner cutting shaft extending through the outer shaft, the inner cutting shaft at least one of translatable or rotatable relative to the outer shaft to cut tissue extending through the window;
a handpiece assembly, including:
a housing;
a drive assembly disposed within the housing and coupled to the inner cutting shaft, the drive assembly configured to drive the at least one of translation or rotation of the inner cutting shaft; and
a trigger coupled to the drive assembly, wherein manual actuation of the trigger actuates the drive assembly to drive the at least one of translation or rotation of the inner cutting shaft; and
an isolation mechanism operably coupled between the proximal hub and the housing to engage the proximal hub and the housing with one another, wherein the isolation mechanism isolates movement of the housing from the end effector assembly through a range of motion.

2. The tissue resecting instrument according to claim 1, wherein the isolation mechanism includes a ball and socket joint.

3. The tissue resecting instrument according to claim 2 wherein one of the proximal hub or the housing includes a concave surface defining a socket of the ball and socket joint, and wherein the other of the proximal hub or the housing includes a convex surface defining a ball of the ball and socket joint, the ball received within the socket.

4. The tissue resecting instrument according to claim 1, wherein the isolation mechanism includes a bellows.

5. The tissue resecting instrument according to claim 4 wherein a proximal end portion of the bellows is coupled to the housing and wherein a distal end portion of the bellows is coupled to the proximal hub.

6. The tissue resecting instrument according to claim 1, further comprising a connector operably coupling the inner cutting shaft and the drive assembly with one another.

7. The tissue resecting instrument according to claim 6, wherein the connector is configured to enable the drive assembly to drive the at least one of translation or rotation of the inner cutting shaft in each of an aligned position, a yawed position, and a pitched position of the housing relative to the proximal hub.

8. The tissue resecting instrument according to claim 1, wherein the handpiece assembly further includes a vacuum generator coupled to the drive assembly and the inner cutting shaft such that, during at least a portion of the manual actuation of the trigger to drive the drive assembly, the vacuum generator generates vacuum to suction cut tissue through the inner cutting shaft and into the vacuum generator.

9. The tissue resecting instrument according to claim 8, wherein the handpiece assembly further includes a tissue collection cartridge configured to releasably engage the housing, the tissue collection cartridge defining a port configured to communicate with the vacuum generator such that, during at least a portion of the manual actuation of the trigger to drive the drive assembly, cut tissue is urged from the vacuum generator through the port into the tissue collection cartridge.
